(19) Europäisches Patentamt
European Patent Office
Office européen des brevets

(11) Veröffentlichungsnummer: **0 132 595**
**B1**

(12) # EUROPÄISCHE PATENTSCHRIFT

(45) Veröffentlichungstag der Patentschrift: **14.06.89**

(51) Int. Cl.⁴: **A 61 K 31/47** // C07D217/20

(21) Anmeldenummer: **84107207.7**

(22) Anmeldetag: **22.06.84**

(54) **Tokolytisches Mittel.**

(30) Priorität: **21.06.83 BG 61406/83**

(43) Veröffentlichungstag der Anmeldung: **13.02.85 Patentblatt 85/7**

(45) Bekanntmachung des Hinweises auf die Patenterteilung: **14.06.89 Patentblatt 89/24**

(84) Benannte Vertragsstaaten: **AT BE CH DE FR GB IT LI LU NL SE**

(56) Entgegenhaltungen:
**CHEMICAL ABSTRACTS, Band 71, 1969, Seite 158, Nr. 29028s, Columbus, Ohio, US; M. KABIR NAIMZADA: "Effect of tetrahydropapaveroline on the rabbit uterus" & ATTI ACCAD. MED. LOMB. 1967, 22(3), 381-4**
**CHEMICAL ABSTRACTS, Band 78, 1973, Seite 16, Nr. 92469w, Columbus, Ohio, US; J.M. MARSHALL u.a.: "Adrenergic influences on uterine smooth muscle" & PHIL. TRANS. ROY. SOC. LONDON, SER. B 1973, 265(867), 135-48**
**CHEMICAL ABSTRACTS, Band 92, 1980, Seite 28, Nr. 87950f, Columbus, Ohio, US; N.S. IVANOVA u.a.: "Bronchospasmolytic activity of triquinol" & FARMATSIYA (SOFIA) 1979, 29(3), 40-6**

**Die Akte enthält technische Angaben, die nach dem Eingang der Anmeldung eingereicht wurden und die nicht in dieser Patentschrift enthalten sind.**

(73) Patentinhaber: **TPO "PHARMACHIM", 16 Ilienско Chaussée, Sofia (BG)**

(72) Erfinder: **Ivanova, Nedyalka Stoyanova, 11, Traicho Kostov Strasse, Sofia (BG)**
Erfinder: **Nikolova, Milka Petrova, Raiko Daskalov Strasse 3, Sofia (BG)**
Erfinder: **Ivanov, Chavdar Borissov, Komplex Mladost-1, Block 46-4, Sofia (BG)**
Erfinder: **Dryanska, Margarita Dimitrova, Komplex Mladost-3, Block 308-3, Sofia (BG)**
Erfinder: **Zabunova, Orhideya Bontcheva, Komplex Mladost-2, Block 215-5, Sofia (BG)**

(74) Vertreter: **von Füner, Alexander, Dr. et al, Patentanwälte v. Füner, Ebbinghaus, Finck Mariahilfplatz 2 & 3 Postfach 95 01 60, D-8000 München 95 (DE)**

**EP 0 132 595 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft die Verwendung eines Tetrahydroisochinolinderivats zur Herstellung von tokolytischen Präparaten, die in der Obstetrik- und gynäkologischen Praxis Anwendung finden.

Es ist bekannt, daß
1-(3,5-Dihydroxyphenyl)-2-[[(4-hydroxybenzyl)-äthyl]-amino]-äthanol Hydrobromid,
aktives Bestandteil des Präparats «Partusisten», ein Arzneimittel mit tokolytischem Effekt darstellt, das beim Einhalten aller Bedingungen fähig ist, alle vorzeitig erscheinenden Gebärmutter-Kontraktionen vollständig zu unterdrücken. Nachteil dieses Präparats sind die Nebenwirkungen auf das Herz-Gefäß-System (1,2,3,4).

Aus C.A. 92, (1980), Nr. 87950f ist die bronchospasmolytische Aktivität von Triquinol bekannt. Bei Triquinol handelt es sich um das
(−)-1,2,3,4-Tetrahydro-1-(3,4,5-trimethoxybenzyl)-6,7-isochiolindiol.

Aus C.A. 71, (1969), Nr. 29028s ist die Wirkung von Tetrahydropapaverolin auf den Kaninchenuterus bekannt, d.h. der myometriumrelaxierende Effekt. Dieser wird mit Dosen von 10 bis 20 µg/kg erreicht.

Die Aufgabe der Erfindung war es nun, ein neues tokolytisches Mittel mit schwächer ausgeprägten Nebenwirkungen auf das Herz-Gefäß-System zu schaffen.

Das Wesen der vorliegenden Erfindung besteht in der Verwendung physiologisch verträglicher Salze eines Tetrahydroisochinolin-Derivats der allgemeinen Formel I

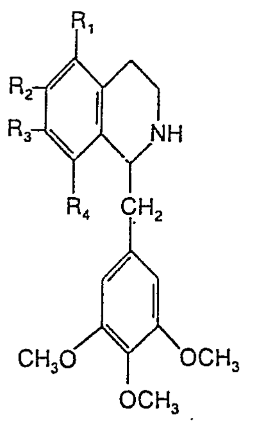

(I)

in der $R_1$ = OH, H, $R_2$ = $R_3$ = OH, H und $R_4$ = OH, H sind, zur Herstellung tokolytischer Mittel in der Obstetrik- und gynäkologischen Praxis.

Es wurde festgestellt, daß das Tetrahydroisochinolin-Derivat der allgemeinen Formel (I) eine unerwartet große und anhaltende tokolytische Aktivität besitzt. Das Tetrahydroisochinolin-Derivat der allgemeinen Formel (I) hemmt (inhibiert) die spontane bioelektrische und Kontraktionsaktivität der Gebärmutter von schwangeren und nichtschwangeren Hasen.

Das erfindungsgemäß verwendete Tetrahydroisochinolin-Derivat der allgemeinen Formel I kann zur Verhinderung einer Fehlgeburtsgefahr, einer vorzeitigen Entbindung und Unterdrückung von Wehen bei einer pathologischen Geburt durch bekannte therapeutische Verfahren in geeigneten therapeutischen Dosen angewandt werden.

Das erfindungsgemäß verwendete Tetrahydroisochinolin-Derivat der allgemeinen Formel (I) kann auch in Form pharmazeutischer Kompositionen wie Tabletten, Injektionslösungen für orale oder parenterale Anwendung sowie auch als Suppositoria angewandt werden.

Die Herstellung der pharmazeutischen Kompositionen wird erfindungsgemäß nach bekannten Verfahren durch Vermischen der aktiven Substanz mit entsprechenden organischen oder anorganischen Hilfsstoffen durchgeführt.

Bei oraler Anwendung beträgt die Dosis der aktiven Substanz von 18 bis 24 mg täglich.

Bei einer optimalen parenteralen Anwendung wird die aktive Substanz in Dosen von 1 bis 5 mkg/min verabreicht.

Bei Suppositoria betragen die täglichen Dosen von 12 bis 16 mg.

Die Vorteile des Isochinolin-Derivats der allgemeinen Formel (I) im Vergleich zu
1-(3,5 Dihydroxyphenyl)-2-[[(1,4-hydroxybenzyl)-äthyl]-amino]-äthanol Hydrobromid A sind folgende:

1. Der inhibierende (hemmende) Effekt des Isochinolin-Derivats der allgemeinen Formel (I) auf die spontane Aktivität bei schwangeren Tieren ist im Vergleich zur Verbindung A besser ausgeprägt, bei welcher der inhibierende Effekt bei schwangeren Tieren eine kürzere Dauer aufweist, als der des Isochinolin-Derivats der allgemeinen Formel (I).

2. Bei der Behandlung schwangerer Tiere mit dem Isochinolin-Derivat der allgemeinen Formel (I) wurde eine Senkung des Basaltonus festgestellt, was für eine starke Relaxation der Gebärmuttermuskulatur spricht. Bei der Behandlung von Tieren mit der Vergleichsverbindung A wird diese Wirkung nicht beobachtet.

3. Der Inhibitionswirkung des Isochinolin-Derivats der allgemeinen Formel (I) tritt im Vergleich zu dieser Verbindung A schneller ein.

4. Beide Verbindungen rufen eine Pulsbeschleunigung bei nichtnarkotisierten Hunden hervor, doch die Zeitdauer der Wiederherstellung der Ausgangsfrequenz des Pulses ist um das Doppelte kleiner beim Isochinolin-Derivat der allgemeinen Formel (I).

5. Bei der Untersuchung des alkalischen Säure-Zustands schwangerer Frauen weist das Isochinolin-Derivat der allgemeinen Formel (I) keine großen Abweichungen in den Hauptkennwerten auf: BE, SB, $pCO_2$, $pO_2$, und es wurde auch keine Tendenz zu Metabolit-Azidose in den ersten Stunden nach seiner Anwendung beobachtet, während bei der Kontroll-Verbindung A solche Abweichungen beobachtet wurden. Diese Vorteile der Verbindung der allgemeinen Formel (I) sind von wesentlicher Bedeutung für den Alkali-Säure-Zustand der Frucht.

6. Die Suppositorium-Anwendungsform des Isochinolin-Derivats der allgemeinen Formel (I) stellt

eine neue wirkungsvollere Form für eine selektive Wirkung der Verbindung auf die Gebärmuttermuskulatur dar.

Die tokolytische Aktivität des Tetrahydroisochinolin-Derivats der allgemeinen Formel (I) und insbesondere der Verbindung $1^a$ $R_2 = R_3 = OH$, $R_1 = R_4 = H$ = 1-1-(3,4,5-Trimethoxybenzyl)-6,7-dihydroxy-1,2,3,4-tetrahydroisochinolin wird anhand der nachstehend angeführten Beispiele näher erläutert.

1. Versuche an Ratten

Vom ovariektomierten und nach dem 17. Tag während vier Tagen einer Behandlung mit Östradiol ausgesetzten Ratten-Weibchen der «Vistar»-Rasse wurden Muskelstreifen vom Uterus Cornis isoliert und in ein Organbad mit temperierter (35 °C) und mit 95% $O_2$ und 5% $CO_2$ belüfteter Krebs-Lösung gebracht.

Die Kontraktionsaktivität wurde über einen mechanoelektrischen Transducer (Energiewandler) Swena Sg 4–90 Gleichstrom-Schreibvorrichtung in isometrischer Betriebsführung registriert.

Das Isochinolin-Derivat $1^a$ und die für den Vergleich benutzte Verbindung A wurden kumulativ während einer spontanen Aktivität, sowie auch während einer durch Oxytozin und Azetylcholin hervorgerufene Aktivität eingeführt. Bei einigen der Versuche wurden kumulative Azetylcholin-Kurven auf der Grundlage beider Präparate entworfen.

Die Analyse der erhaltenen Resultate zeigt, daß die Verbindung $1^a$ eine sehr gut ausgeprägte inhibierende Wirkung sowohl auf die spontane Aktivität des myometrischen (Fig. 1) als auch auf die durch Oxytozin oder durch Azetylcholin hervorgerufenen Kontraktionen (des Myometriums) (Fig. 2) aufweist.

Die inhibierende Wirkung der Verbindung $1^a$ auf die spontane Kontraktionsaktivität tritt bei Kontraktionen von $1{,}10^{-16}$ und $1{,}10^{-8}$ Mol ein, indem eine volle Inhibierung der durch einen spezifischen Stimulator Oxytozin hervorgerufenen Kontraktionen bei Konzentrationen von $1{,}10^{-7}$ Mol beobachtet wird. Das vorher eingeführte Propanol beseitigt die inhibierende Wirkung der Verbindung.

Das Isochinolin-Derivat $1^a$ und die zum Vergleich benutzte Verbindung A rufen ein wesentliches Verschieben der kumulativen Kurven des Azetycholins nach rechts, welche dem Verlauf nach übereinstimmen.

Wenn das Azetylcholin auf der Grundlage von $1^a$ in größeren Konzentrationen ($1{,}10^{-9}$ Mol) eingeführt wird, beobachtet man eine krasse Verminderung der kontraktiven Antwort gegenüber dem Azetylcholin (Fig. 3), weswegen es nicht möglich ist, eine kumulative Kurve zu entwerfen.

2. Versuche an wachen Hunden

Die Versuche wurden mit wachen Hunden gemäß dem von Milenov und Mitarbeitern beschriebenen Verfahren durchgeführt (2).

a) Wirkung des
1-1-(3,4,5-Trimethoxybenzyl)-6,7-dihydroxy-1,2,3,4-tetrahydroisochinolin Hydrochlorids $1^a$ auf das Mechanogramm und myoelektrische Aktivität der Gebärmutter.

Es wurde die spontane Aktivität der Gebärmutter aufgetragen, die aus Spikegruppen mit einer Dauer von 1 bis 1,5 Minuten (die sogenannten langen Spikegruppen) besteht, welche in einem Intervall von 15 bis 60 Sekunden erscheinen (Fig. 4). Im Schritt der Spikegruppen wurde auch die Kontraktionsaktivität registriert, welche in diesem Fall die Änderungen des Gebärmutter-Innendrucks wiedergibt (Fig. 4).

Bei der Einführung von Dosen von 1 mg/kg der Verbindung $1^a$ wird eine totale Inhibierung der myoelektrischen und der Kontraktionsaktivität während 3 bis 6 Minuten beobachtet. Danach folgte eine Phase allmählicher Wiederherstellung (Rehabilitation) in der achten bis zehnten Minute. Die Vergrößerung der Dosis auf 2 mkg/kg hatte eine totale Inhibierung sowohl der Kontraktions-, als auch der Spikeaktivität mit einer Dauer von 8 bis 10 Minuten zufolge (Fig. 5). Dieser Inhibierung folgt eine Phase fortschreitender Wiederherstellung der Ausgangsaktivitäten, wobei die endgültige Rehabilitation zwischen der 12. bis 15. Minute eintritt. Analoge Wirkungen wurden auch nach der Einführung der Verbindung A beobachtet, doch mit dem Unterschied, daß der Effekt des Derivats $1^a$ im Vergleich zu dem der Verbindung A schneller eintritt (Fig. 6).

3. Die Wirkung des
1,1-(3,4,5 Trimethoxybenzyl)-6,7-dihydroxy-1,2,3,4-tetrahydroisochinolin Hydrochlorids $1^a$ auf die durch Oxytozin stimulierte zusammenziehende und myoelektrische Aktivität der Gebärmutter

Die intravenöse Einführung von Oxytozin in einer Dosis von 0,06 ME/kg ruft eine krasse Zunahme des Tonus und der Amplitude in dem Mechanogramm hervor. In dem Elektrouteromyogramm drückte sich die Oxytozin-Wirkung in einer fortlaufenden Spikeaktivität in den ersten 2 bis 3 Minuten aus, welche später von kurzen Ruhepausen mit einer Dauer von 5 bis 10 Sekunden unterbrochen wurde (Fig. 7). Bei unseren Versuchen ließ die Oxytozin-Wirkung auf die Gebärmutter in 30 bis 40 Minuten nach der Einführung nach.

Auf dieser Grundlage eingeführt, inhibiert die Verbindung $1^a$ in einer Dosis von 2 mkg/kg deutlich die zusammenziehende und Spikeaktivität bei allen Versuchen (Fig. 7).

4. Die Wirkung des
1-1-(3,4,5-Trimethoxybenzyl)-6,7-dihydroxy-1,2,3,4-tetrahydroisochinolin Hydrochlorids $1^a$ auf das Verhalten der Tiere und die Pulsfrequenz.

Die erstmalige Einführung der Verbindung $1^a$ und der Vergleichsverbindung A rufen eine Beschleunigung des Pulses in den ersten Minuten nach deren Einführung hervor. Diese Wirkung läßt nach 30 bis 40 Minuten im Fall der Verbindung $1^a$ und in 70 bis 80 Minuten im Fall der Verbindung A völlig nach. Jede folgende Injizierung des Derivats $1^a$ sogar in größeren Dosen ruft eine schwächere Wirkung auf die Pulsfrequenz hervor. Bei diesen Dosen wurden keine Veränderungen im Verhalten der Tiere beobachtet.

5. Versuche an Hasen

Zu diesem Zweck wurden an nichtschwangeren und schwangeren Häsinnen (nach dem 20sten Tag) gemäß dem von Milenov und Mitarbeitern (3) beschriebenen Verfahren, auf der Grundlage einer spontanen Aktivität Versuche durchgeführt.

Die Analyse der erhaltenen Daten zeigte, daß das Derivat 1[a] in Dosen von 1 mkg/kg die myoelektrische Aktivität sowohl bei nichtschwangeren (Fig. 8) als auch bei schwangeren Tieren (Fig. 9) hemmt.

Das Isochinolin-Derivat 1[a] inhibiert die Tonuskontraktionen und die auf denen superponierten Phasenkontraktionen. Gleichzeitig wurde bei den schwangeren Tieren auch eine Verminderung des Basaltonus beobachtet, was für eine starke Relaxation der Gebärmuttermuskulatur spricht.

Analoge inhibierende Wirkungen wurden ebenso nach dem Einführen der Verbindung A beobachtet. In diesem Fall ist aber die inhibierende Wirkungsdauer bei den schwangeren Tieren verhältnismäßig kürzer im Vergleich zu der der Verbindung 1[a]. Andererseits wurde keine Verminderung des Basaltonus beobachtet, wie es bei der Einführung der Verbindung 1[a] an schwangere Tiere der Fall ist (Fig. 10).

6. Klinische Versuche

Das Isochinolin-Derivat 1[a] wurde an 53 Patientinnen als tokolytisches Mittel bei spontanen Fehlgeburten und vorzeitigen Entbindungen geprüft.

Zu Beginn der Behandlung wurde das Isochinolin-Derivat 1[a] venös als eine venöse Tropf-Infusion mit Glukose angewandt, um schnell die Gebärmutterkontraktionen zu mildern, wonach zu einer peroralen Verabreichung von Tabletten oder zu Suppositoria für anale Anwendung übergegangen wurde.

Der Effekt der angewandten unterschiedlichen Therapie-Formen wurde durch eine Monitorkontrolle der Gebärmutterkontraktionen und der Kinder-Herztöne, durch eine dynamische Beobachtung des Alkali-Säure-Status (ASS) der schwangeren Frauen, durch die Beobachtung einiger biochemischer Kennwerte für den Zustand der Schwangeren (SMA 7 und SMA 12), ECG, Blutdruck, auf die Verträglichkeit des Präparats und anamnestische Daten der Patientin über die Wirkung des Präparats auf das Schmerzsyndrom geschätzt. Beim Vergleich des Isochinolin-Derivats 1[a] und der Kontroll-Verbindung A im Hinblick auf den inhibierenden Effekt der vorzeitigen Kontraktilität bei schwangeren Frauen wurde festgestellt, daß in den mit der Verbindung 1[a] behandelten Fällen 88% als erfolgreich bezeichnet wurden, während diese Fälle, bei denen die Verbindung A angewandt wurde, sich 85% als erfolgreich erwiesen haben.

Bei der Kontrolle des Alkali-Säure-Zustands der mit der Verbindung 1[a] in anhaltender venöser Infusion behandelten schwangeren Frauen wurden keine großen Abweichungen in den Hauptkennwerten: $pO_2$, $pCO_2$, SB, BE und pH-Wert (Tabelle 1) festgestellt. Es wurde auch keine Tendenz zu einer Metabolyt-Azidose festgestellt, demgegenüber solche Abweichungen bei der Behandlung mit der Kontroll-Verbindung A beobachtet werden, demzufolge die letztere eine Stabilisierung mit Chromäthanol erfordert (Tabelle 2).

Die Verbindung 1[a] wird von den Patientinnen sehr gut vertragen, subjektive Abweichungen klingen verhältnismäßig schnell ab, wobei die Nebenerscheinungen (Beschleunigung der Herzaktivität) nicht allzu stark ausgeprägt sind.

Ein ungünstiger Einfluß auf die Herzfrequenz des Fötus wurde nicht festgestellt.

Die Verbindung 1[a] wies keine schädliche Wirkung auf die geborenen Kinder auf, sowie auch auf die Nichtgeborenen, was mit Hilfe eines Monitors ermittelt wurde.

Es wurde eine bessere Inhibierung des Schmerz-Syndroms und der vorzeitigen Kontraktionen bei der Anwendung des Präparats als Suppositorium anstatt als Tabletten festgestellt.

Anhand der nachstehend angeführten Beispiele wird die Herstellung der fertigen Arzneiformen näher erläutert.

Beispiel 1

Herstellung der Injektionslösungen

Ingredienzien für die Herstellung von 10 l Injektionslösung.

| | |
|---|---|
| 1-1-(3,4,5-Trimethoxybenzyl)-6,7-dihydroxy-1,2,3,4-tetrahydroisochinolin Hydrochlorid 1[a] | 0,5 g |
| Askorbinsäure 99% | 2,0 g |
| Natriumsulfit wasserfrei 100% | 1,0 g |
| Natriumchlorid 99,5% | 85,0 g |
| Wasser für Injektionen bis zu | 10,0 g |

Frisch destilliertes Wasser für Injektionen wird während 15 bis 20 Minuten gekocht, wonach es durch Barbotage mit reinem Stickstoff bis zur Abkühlung gesättigt wird. In cirka 9 Liter von dem auf diese Weise gesättigten Wasser werden nacheinanderfolgend mittels Schütteln des Natriumsulfit, das Natriummetabisulfit, die Askorbinsäure, das Natriumchlorid und die aktive Komponente bei unterbrochener Sättigung mit Stickstoff aufgelöst. Die fertige Lösung wird durch «Minipor» mit einer Membrane 0,45 μ unter Stickstoff filtriert. Das Füllen der Ampulle erfolgt unter doppeltem Begasen mit Stickstoff. Die Ampullen werden unter strömendem Wasserdampf bei 100 °C während 30 Minuten sterilisiert.

Beispiel 2

Herstellung von Suppositoria

Beispielsweise Zusammensatzung:

| | |
|---|---|
| 1-1-(3,4,5-Trimethoxybenzyl)-6,7-dihydroxy-1,2,3,4-tetrahydroisochinolin 1[a] | von 0,1 bis 0,5% |
| Vehiculum (Trägersubstanz) | bis zu 100% |

Die aktive Komponente wird gemahlen und mit einem indifferenten Füllstoff vermischt. Das Vehiculum wird geschmolzen. Mit dem pulverartigen

Gemisch und einem Teil des geschmolzenen Vehiculums wird ein primäres Gemisch erhalten, das mit dem restlichen Vehiculum verdünnt wird. Das Gemisch wird anschließend bis zur Homogenisierung vermengt und einem Automat für die Formung der Suppositoria zugeführt.

Beispiel 3
Herstellung von Tabletten

Beispielsweise Zusammensetzung:

| | |
|---|---|
| 1-1-(3,4,5-Trimethoxybenzyl)-6,7-dihydroxy-1,2,3,4-tetrahydroisochinolin Hydrochlorid 1[a] | 0,003 g |
| Milchzucker (Laktobiose) | 0,035 g |
| Weizen-Stärkemehl | 0,018 g |
| Talk | 0,002 g |
| Magnesiumstearat | 0,001 g |
| Gelatine | 0,001 g |
| | 0,060 g |
| Filmüberzug | 0,003 g |

Die aktive Komponente, der Milchzucker und ein Teil des Stärkemehls wird mit einer 10% Gelatine-Lösung granuliert. Das erhaltene Granulat wird bis zu einer Restfeuchtigkeit von 2 bis 3% getrocknet, mit dem restlichen Stärkemehl, Talk und Magnesiumstearat bepudert und tablettiert. Die hergestellten Tabletten werden mit einem Film aus Lack auf Azetonbasis überzogen.

Tabelle 1
Mittelwerte und Standard-Abweichungen der Alkali-Säure-Kennwerte und der Blutgase bei schwangeren Frauen im Fall einer anhaltenden Infusion mit dem Isochinolin-Derivat 1a. Anzahl der Fälle: 12

| Faktoren | Ausgangsdaten | erste Stunde | zweite Stunde | dritte Stunde | vierte Stunde | zehnte Stunde |
|---|---|---|---|---|---|---|
| pH | 7,45 ± 0,04 | 7,44 ± 0,01 | 7,43 ± 0,03 | 7,44 ± 0,02 | 7,44 ± 0,02 | 7,45 ± 0,01 |
| BE | −6,1 ± 1,9 | −6,7 ± 2,4 | −7,9 ± 2,7 | −6,3 ± 2,6 | −6,2 ± 2,6 | −6,3 ± 3,00 |
| SB | 19,6 ± 1,2 | 20,2 ± 2,3 | 18,5 ± 2,0 | 19,7 ± 1,9 | 19,4 ± 1,9 | 19,4 ± 2,4 |
| $pCO_2$ | 23,0 ± 2,5 | 23,2 ± 2,4 | 22,4 ± 3,00 | 23,2 ± 3,2 | 23,3 ± 3,6 | 23,2 ± 3,5 |
| $pO_2$ | 84,4 ± 8,9 | 90,4 ± 17,8 | 96,0 ± 21,2 | 89,8 ± 20,3 | 82,2 ± 9,9 | 82,4 ± 14,5 |

Tabelle 2
Mittelwerte und Standard-Abweichungen der Alkali-Säure-Kennwerte und der Blutgase bei schwangeren Frauen im Fall einer anhaltenden Infusion mit der Kontroll-Verbindung A. Anzahl der Fälle: 20

| Faktoren | Ausgangsdaten | erste Stunde | zweite Stunde | dritte Stunde | vierte Stunde |
|---|---|---|---|---|---|
| pH | 7,44 ± 0,04 | 7,41 ± 0,04 | 7,43 ± 0,05 | 7,43 ± 0,03 | 7,42 ± 0,04 |
| BE | −4,3 ± 3,3 | −6,6 ± 3,2 | −6,1 ± 3,00 | −6,00 ± 3,1 | −4,0 ± 3,4 |
| SB | 21,8 ± 3,9 | 19,1 ± 3,1 | 19,5 ± 2,2 | 19,7 ± 2,2 | 20,3 ± 2,8 |
| $pCO_2$ | 27,0 ± 6,3 | 23,9 ± 5,6 | 25,4 ± 5,4 | 26,0 ± 6,1 | 25,2 ± 5,7 |
| $pO_2$ | 85,8 ± 11,2 | 84,2 ± 8,9 | 83,0 ± 10,1 | 83,4 ± 9,4 | 81,1 ± 10,5 |

Die beigefügten Abbildungen zeigen im einzelnen:

Fig. 1: Wirkung des 1-1-(3,4,5-Trimethoxybenzyl)-6,7-dihydroxy-1,2,3,4-tetrahydroisochinolin Hydrochlorids und der Verbindung A auf die spontanen Kontraktionen des Myomestralstreifens einer Ratte.

Fig. 2: Wirkung des 1-1-(3,4,5-Trimethoxybenzyl)-6,7-dihydroxy-1,2,3,4-tetrahydroisochinolin Hydrochlorids und der Verbindung A auf die durch Oxytozin hervorgerufene kontraktive Aktivität des Myometralstreifens.

Fig. 3: Wirkung des 1-1-(3,4,5-Trimethoxybenzyl)-6,7-dihydroxy-1,2,3,4-tetrahydroisochinolin Hydrochlorids ($1.10^{-9}$) auf die kontraktile Antwort des Myometralstreifens gegenüber Azetylcholin.

Fig. 4: Elektrische und kontraktive Aktivität des Uterus einer Hündin in den Verhältnissen eines chronischen Versuches. Die elektrische Aktivität und der Gebärmutter-Innendruck sind mit unterschiedlicher Geschwindigkeit aufgezeichnet.

Fig. 5: Elektrische (Ableitung 1, 2 und 3) und kontraktive Aktivität des Uterus einer Hündin vor und nach dem Injizieren mit 1-1-(3,4,5-Trimethoxybenzyl)-6,7-dihydroxy-1,2,3,4-tetrahydroisochinolin Hydrochlorid. Es ist nach einer Pause von 9 Minuten die Wiederherstellung der elektrischen Aktivität demonstriert.

Fig. 6: Elektrische (Ableitung 1, 2 und 3) und kontraktive Aktivität des Uterus einer Hündin vor und nach dem Injizieren mit der Verbindung A. Es ist nach einer Pause von 9 Minuten die Wiederherstellung der elektrischen Aktivität demonstriert.

Fig. 7: Elektrische (Ableitungen 1, 2, 3 und 4) und kontraktive Aktivität durch Oxytozin hervorgerufen. Das Injizieren von 1-1-(3,4,5-Trimethoxybenzyl)-6,7-dihydroxy-1,2,3,4-tetrahydroisochinolin

Hydrochlorid 1a auf dieser Grundlage weist einen inhibierenden Effekt auf.

Fig. 8: Elektrische (Ableitungen 1 und 3) und kontraktive Aktivität des Uterus einer nichtschwangeren Häsin vor und nach dem Injizieren von 1-1-(3,4,5-Trimethoxybenzyl)-6,7-dihydroxy-1,2,3,4-tetrahydroisochinolin Hydrochlorid 1a.

Fig. 9: Elektrische (Ableitungen 1, 7 und 9) und kontraktive Aktivität des Uterus einer schwangeren Häsin vor und nach dem Injizieren mit 1-1-(3,4,5 Trimethoxybenzyl)-6,7-dihydroxy-1,2,3,4-tetrahydroisochinolin

Hydrochlorid. Es wird nach einer Pause von 10 Minuten eine unvollständige Wiederherstellung der elektrischen Aktivität demonstriert.

Fig. 10: Elektrische (Ableitungen 1, 7 und 9) und kontraktive Aktivität des Uterus einer schwangeren Häsin vor und nach dem Injizieren der Verbindung A. Es wird nach einer 10-minütigen Pause eine vollständige Wiederherstellung der elektrischen und kontraktiven Aktivität demonstriert.

LITERATURFUNDSTELLEN:

1. The action of sympathicomimetic drugs on uterine muscle, F.K. Klöck – «Labour inhibition betamimetic drugs in obstretrics», p. 27, 1977
2. The action of betamimetics on the myometrium, R. Czekanowski; – «Labour inhibition betamimetic drugs in obstretrics», p. 11, 1977
3. The administration of beta-sympathomimetic agents in obstretric emergencies, F. Ropke, H.Y. Woraschk.; – «Labour inhibition betamimetic drugs in obstretrics», p. 235, 1977
4. Current status of tocolysis labour inhibition; Hans Weidinger; – «Labour inhibition betamimetic drugs in obstretrics», p. 243, 1977
5. Trikvinol – experimental and clinical tests, Collection «Pharmachim»-Bulgaria
6. Japanese patent Nr. 27864/1968
7. English patent Nr. 1173719/1969

**Patentanspruch**

Verwendung eines Tetrahydroisochinolinderivats der allgemeinen Formel I

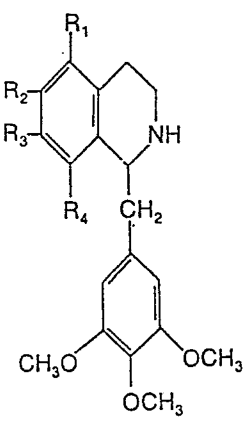

(I)

in der $R_1$ = OH, H, $R_2$ = $R_3$ = OH, H, $R_4$ = OH, H sind, zur Herstellung von tokolytischen Präparaten.

**Claim**

Use of a tetrahydroisoquinoline derivative of the general formula I

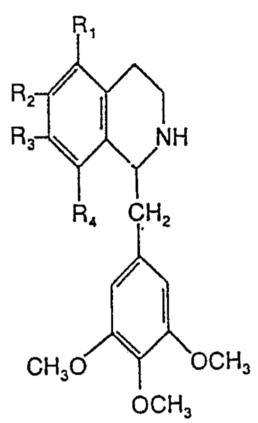

(I)

in which $R_1$ = OH, H, $R_2$ = $R_3$ = OH, H, $R_4$ = OH, H, for the production of tocolytic preparations.

**Revendication**

Utilisation d'un dérivé de tétrahydroisoquinoléine, représenté par la formule générale (I):

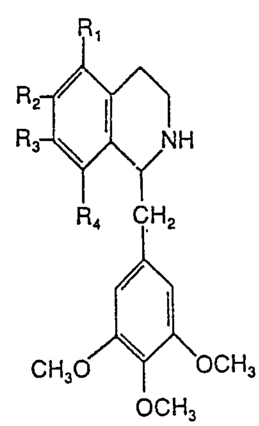

(I)

dans laquelle:
- $R_1$ = OH, H;
- $R_2$ = $R_3$ = OH, H;
- $R_4$ = OH, H,

pour la fabrication de préparations tocolytiques.

Fig. 1

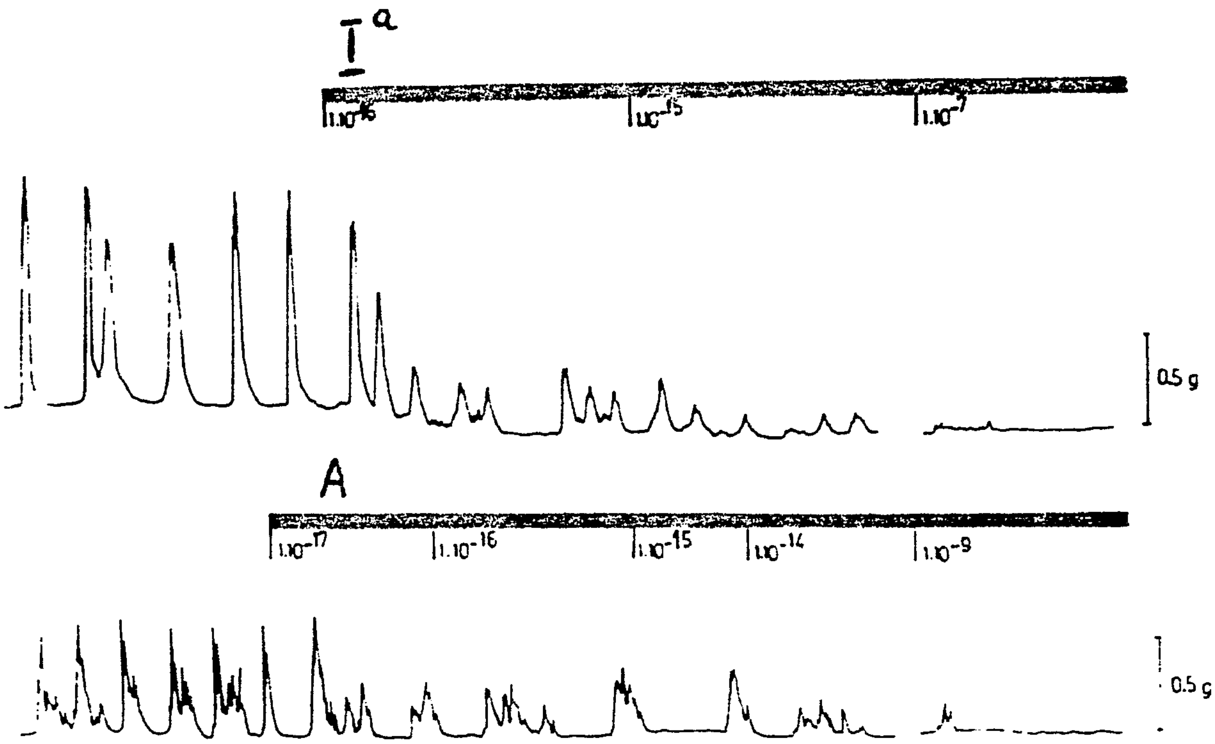
I a
1.10-16
1.10-15
1.10-7
0.5 g
A
1.10-17
1.10-16
1.10-15
1.10-14
1.10-9
0.5 g

Fig. 2

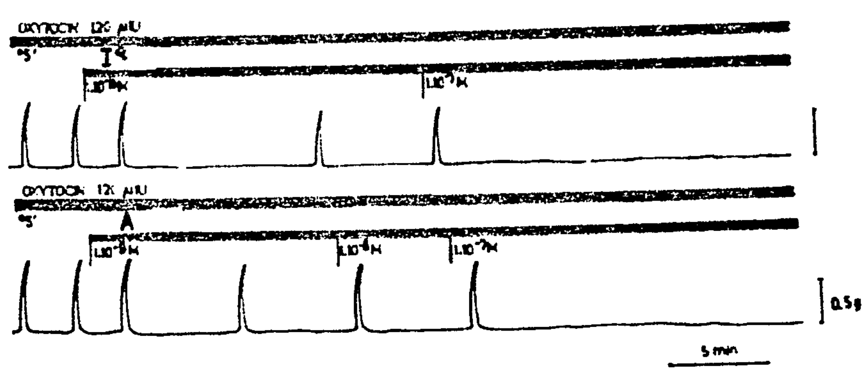
OXYTOCIN 12X µIU
I a
OXYTOCIN 12X µIU
A
0.5 g
5 min

Fig. 3

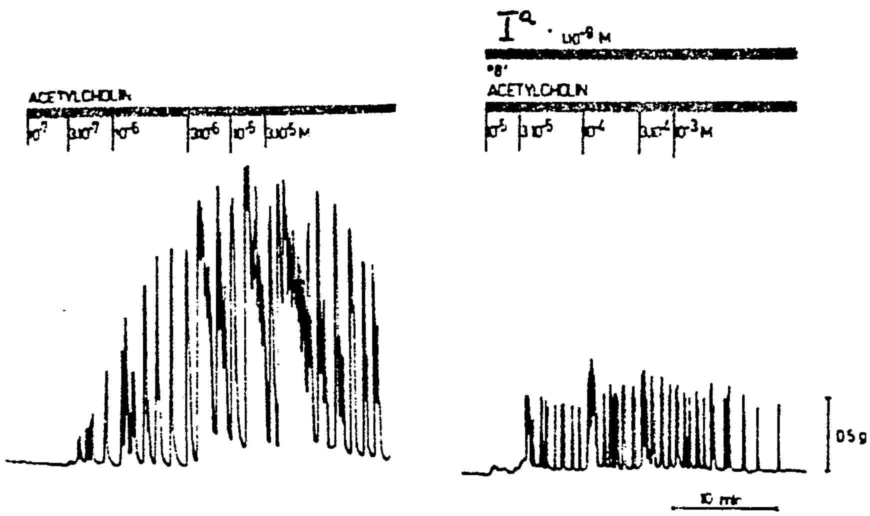
ACETYLCHOLIN
ACETYLCHOLIN
10 min
0.5 g

Fig. 4

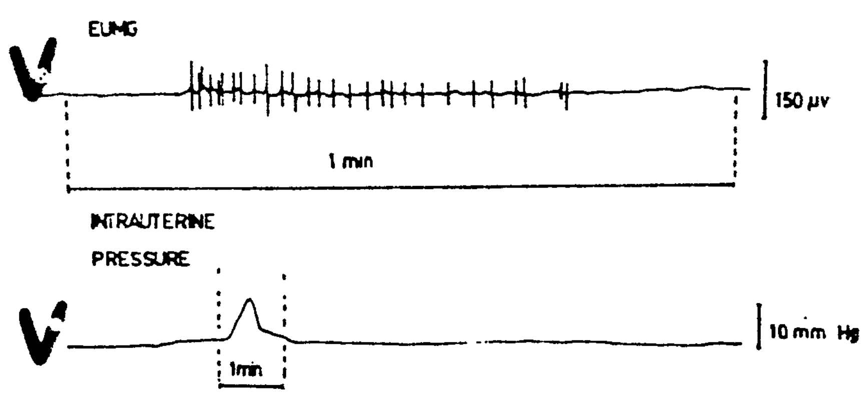
EUMG
150 µv
1 min
INTRAUTERINE
PRESSURE
10 mm Hg
1min

Fig. 5

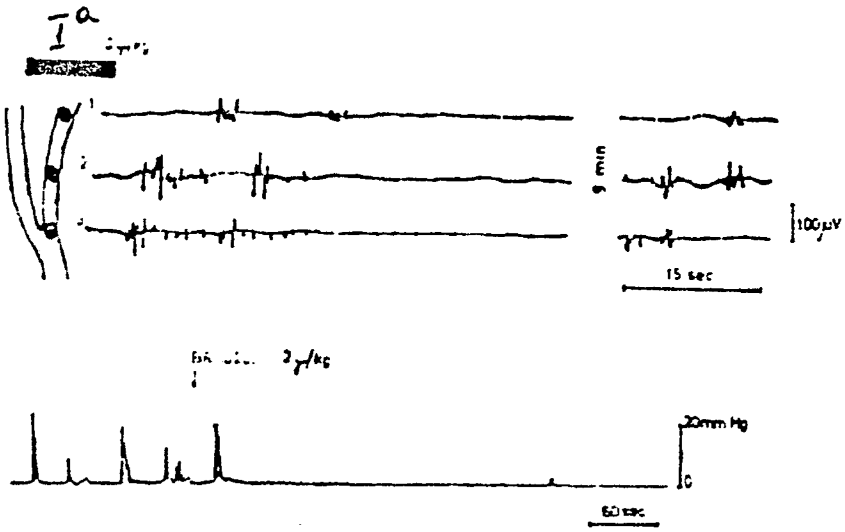
1
2
3
100μV
15 sec
20mm Hg
0
60 sec

Fig. 6

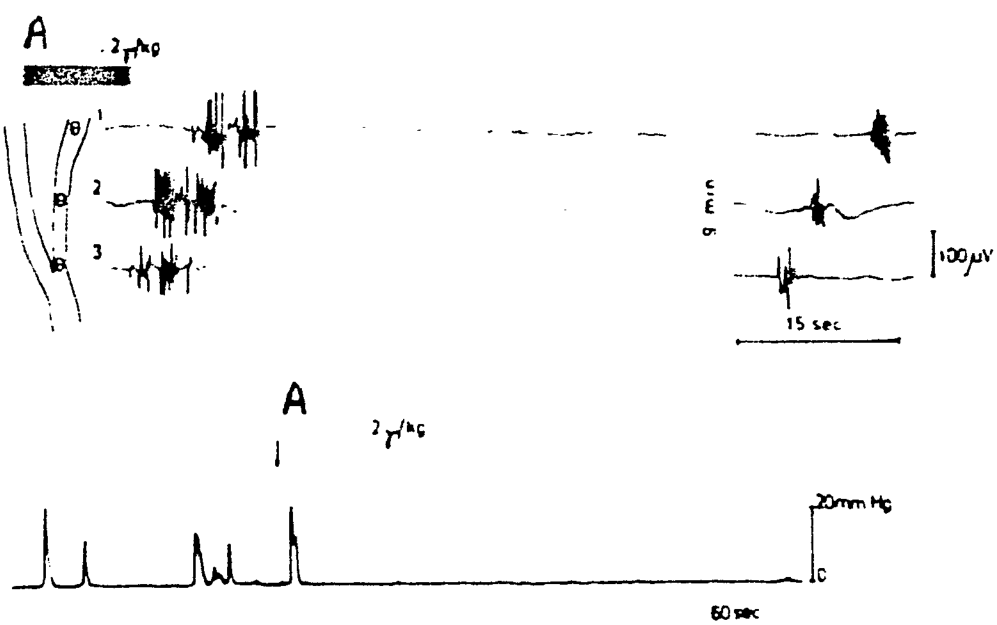
A
1
2
3
100μV
15 sec
A
20mm Hg
0
60 sec

Fig. 7

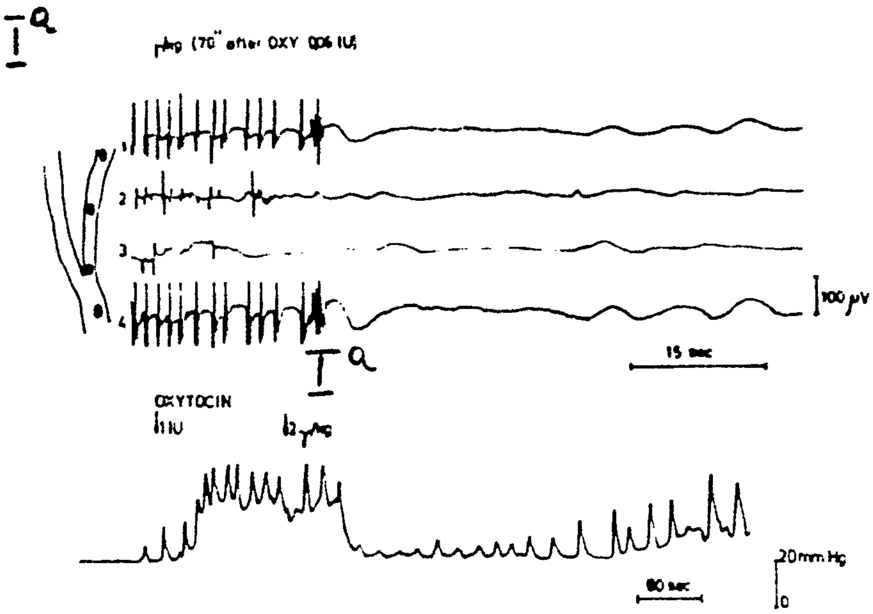
OXYTOCIN
100 μV
15 sec
20 mm Hg
0

Fig. 8

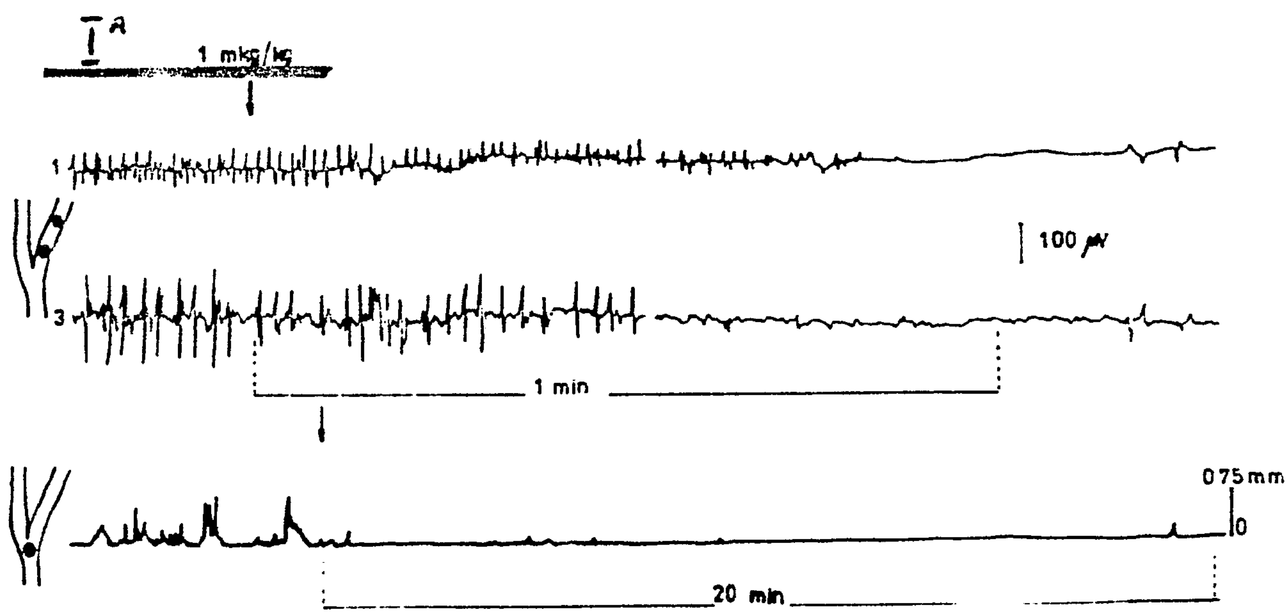
1 mkg/kg
100 μV
1 min
20 min
0.75 mm
0

Fig. 9

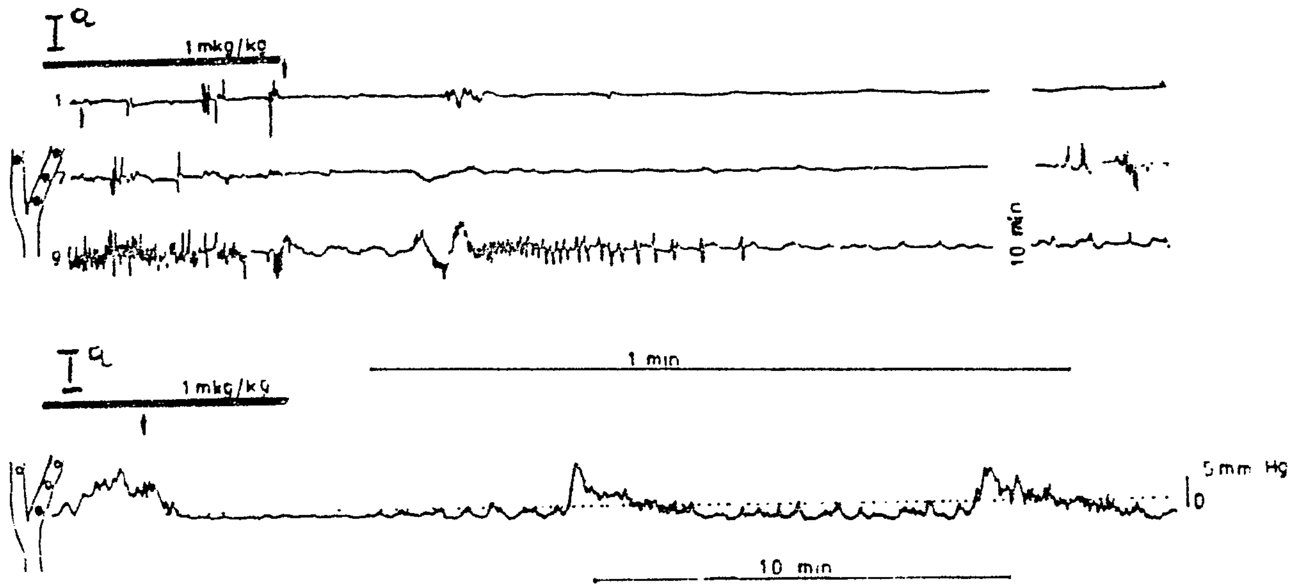
I a
1 mkg/kg
1
9
10 min
I a
1 mkg/kg
1 min
5 mm Hg
0
10 min

Fig. 10

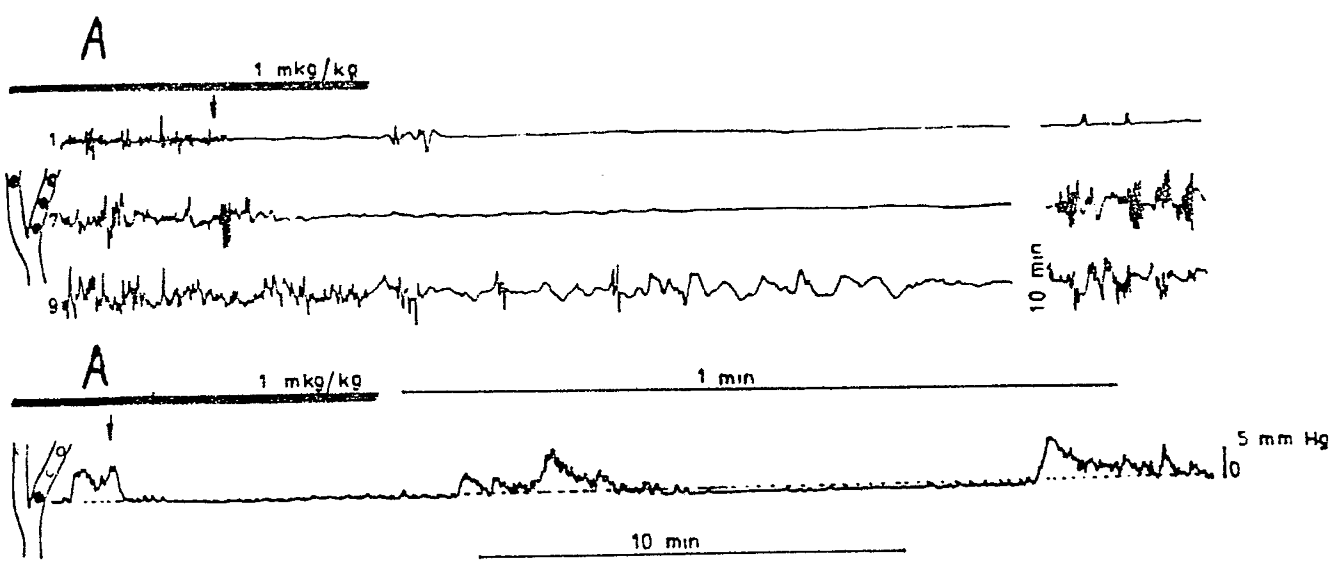
A
1 mkg/kg
1
7
9
10 min
A
1 mkg/kg
1 min
5 mm Hg
0
10 min